# EUROPEAN PATENT SPECIFICATION

(11) **EP 4 316 344 B1**
(45) Date of publication and mention of the grant of the patent: **09.07.2025**
(21) Application number: 22904501.8
(22) Date of filing: 15.11.2022
(51) Int. Cl.: A61B 1/005, A61B 1/00, A61B 1/05, A61B 1/06

(54) **ENDOSCOPE CONTROLLED BY POWER RECEPTOR**
ENDOSKOP MIT STEUERUNG DURCH LEISTUNGSREZEPTOR
ENDOSCOPE COMMANDÉ PAR UN RÉCEPTEUR D'ÉNERGIE

(30) Priority: 08.12.2021 KR 20210174907
(43) Date of publication of application: 07.02.2024
(73) Proprietor: Medintech Inc., Seoul 03100 (KR)
(72) Inventor: LEE, Chi Won, Namyangju-si, Gyeonggi-do 12249 (KR); KIM, Myung Joon, Gwacheon-si Gyeonggi-do 13831 (KR); KOH, Suk Gyu, Seoul 03066 (KR)
(74) Representative: RGTH
(86) International application number: PCT/KR2022/017936
(87) International publication number: WO 2023/106668

(56) References cited:
- WO-A1-2021/145051
- JP-A- 2002 325 724
- JP-A- H05 309 066
- JP-A- H05 329 097
- KR-A- 20150 030 949
- KR-A- 20210 031 231
- KR-B1- 102 284 135
- KR-B1- 102 443 789
- US-A1- 2015 080 658

## Description

### TECHNICAL FIELD

The present disclosure relates to an endoscope controlled by a power receiving part and, more particularly, to an endoscope, the movement of which is controlled by a power receiving part configured to receive power provided by a power source.

### BACKGROUND ART

The information disclosed in this section is only provided for an understanding of background information of embodiments of the present disclosure and should not be taken as a description of the prior art.

An endoscope generally refers to a medical instrument for examining the interior of the body for medical purposes. Such an endoscope may be referred to as "bronchoscope," "gastric endoscope," "laparoscope," or "colonoscope" depending on the area to be examined therewith. Unlike most medical imaging devices, the endoscope is a medical device which is inserted directly into the body.

Due to the development of optical fibers and the rapid development of optical technology and electronics, endoscope technology has reached the stage of the current electronic endoscope and has made a great contribution to the development of the field of gastroenterology. With the development of the electronic endoscope, the electronic endoscope is used not only in the diagnostic field to directly look into and perform histological examinations of a subject's body, but may also replace invasive surgery due to the rapid development of various treatment endoscopes.

The configuration of the endoscope may generally include an insertion tube configured to be inserted into the patient's body with a bendable section and a flexible portion, a control body connected to one end of the insertion tube to control the bending motion of the bendable section, a connector coupled to a light source device, or the like, and a universal code spacing the control body and the connector apart from each other.

The endoscope has a structure in which a mechanical cord (or cable) is disposed between the bendable section and the control body to control the bending motion of the bendable section and the mechanical cord is connected to a control knob disposed on the control body. According to the structure of the endoscope, when the user, a doctor, manually operates the control knob, the mechanical cord transmits power to enable the bending motion of the bendable section.

While a doctor is performing an endoscope, an emergency situation fatal to the patient may occur. However, such an endoscope having the above-described structure requires a doctor to control the bending motion of the bendable section by manually operating the knob in the emergency situation, thereby causing the doctor to only focus on operating the knob. Thus, it may be difficult to overcome the emergency situation, and the bending motion of the bendable section may not be accurately performed.

Therefore, there was developed an endoscope in which the bending motion of a bendable section may be performed automatically using a power source rather than by a manual operation of a doctor. However, the endoscope with this structure may have control precision and stability problems due to the absence of an appropriate structure able to receive power from the outside of the endoscope.

Therefore, there is an urgent need to develop a connector having a novel structure by which the endoscope may receive power from an external source and efficiently transmit the same to a bendable section while maintaining precision and stability in bending control.

The information disclosed in the Background section is technical information that the inventors possessed for, or acquired during, derivation of embodiments of the present disclosure and should not be taken as known technology disclosed to the public before the filing of the embodiments of the present disclosure.

US 2015/080658 A1 discloses an endoscope device and a method of operating the endoscope device. The endoscope device includes: a knob driving part configured to be driven in a mode selected by an operator from either of an automatic mode and a manual mode; a bending part connected to the knob driving part and configured to change a position of a distal end of the endoscope device by driving of the knob driving part, a power generating part configured to provide power to the knob driving part; and a power transmitting part configured to drive the power generating part according to a manipulation of the knob driving part.

WO 2021/145051 A1 discloses an endoscope system, which outputs a state of use of an endoscope by users, includes the endoscope and a processor, wherein: the endoscope has at least two bending modes and has an operating part which can select the at least two bending modes; and the processor acquires a case identifier corresponding to a case for which the endoscope is used, generates use state information pertaining to the used bending mode, and outputs the result by associating the case identifier with the use state information.

JP H05 309066 A discloses an endoscope device is provided with a curved part provided in an inserting part, a curvature wire for curving the curved part by drawing it, a rotating body which is provided in an operating part and drives the curvature wire , motors which are provided in the outside of the operating part and become driving sources for driving the rotating body, and curvature wires for transferring driving force by the motors to the rotating body.

### DISCLOSURE

### Technical Problem

Accordingly, the present disclosure has been made in consideration of the above-described problems occurring in the related art, and the present disclosure provides an endoscope configured to control the movement of a bendable section while maintaining precision and stability in bending motions by controlling the travel distance of a power receiving part configured to receive power from an external source.

The objectives of the present disclosure are not limited to the aforementioned descriptions, and other objectives not explicitly disclosed herein will be clearly understood by a person having ordinary knowledge in the art from the description provided hereinafter.

### Technical Solution

According to the invention as defined in independent claim 1, an endoscope includes a power receiving part configured to move by receiving power from a power source; and a bendable section, a bending motion of which is controlled by a movement of the power receiving part.

In some embodiments, the bendable section may include an imaging means configured to be inserted into a subject's body to collect image information and an illumination means configured to illuminate an interior of the subject's body.

In some embodiments, the endoscope may further include a control body configured to generate a control signal, wherein the control signal generated by the control body controls rotational force of the power source.

In some embodiments, the endoscope may further include a control body configured to generate a control signal, wherein the control signal generated by the control body controls the travel distance of the power receiving part.

In some embodiments, the power receiving part may include a first slider and a second slider, and the control signal may include a first control signal to control a travel distance of the first slider and a second control signal to control a travel distance of the second slider.

In some embodiments, the power receiving part may include a pair of power receiving parts, wherein one of the pair of power receiving parts controls upward and downward bending of the bendable section and the other of the pair of power receiving parts controls left and right bending of the bendable section.

In some embodiments, power generated due to the movement of the power receiving part may be transmitted to the bendable section through the control body.

In some embodiments, the power receiving part receives power directly from an external device including the power source therein. In some embodiments, the power receiving part may receive power directly from a light source device including the power source therein or an image processing device including the power source therein.

According to another aspect of the present disclosure, an endoscope may include one or more among: an insertion tube including a bendable section configured to be inserted into a subject's body to collect image information; a control body configured to generate a control signal to control a bending angle of the bendable section; and a connector including an output part configured to output the control signal and a receiving part configured to receive external power corresponding to the control signal, wherein the control signal controls a travel distance of the receiving part.

### Advantageous Effects

According to embodiments of the present disclosure as described above, provided is the endoscope configured to control the movement of a bendable section while maintaining precision and stability in bending motions by controlling the travel distance of a power receiving part configured to receive power from an external source.

In addition, the present disclosure has a variety of effects with excellent versatility depending on the embodiment, and such effects may be clearly understood from the following description of embodiments.

### DESCRIPTION OF DRAWINGS

The following drawings accompanying the specification illustrate embodiments of the present disclosure and, together with the foregoing disclosure, serve to provide further understanding of the technical spirit of the present disclosure, and thus, the present disclosure should not be construed as being limited to the drawings.
FIG. 1 illustrates the shape of an endoscope according to an embodiment of the present disclosure.
FIG. 2 illustrates the shape of the connector of the endoscope according to an embodiment of the present disclosure.
FIG. 3 illustrates the power receiving part and the power transmission part as the internal shape of the connector of FIG. 2 from which the cover is removed.
FIG. 4 illustrates the shape of a light source device to which the endoscope according to an embodiment of the present disclosure is coupled.
FIG. 5 illustrates a combined shape of the power providing part disposed on the light source device of FIG. 4 and the power receiving part disposed on the connector of FIG. 3.

### MODE FOR INVENTION

Advantages and features of the present disclosure, as well as methods of realizing the same, will be more clearly understood from the following detailed description of embodiments when taken in conjunction with the accompanying drawings. However, the present disclosure is not limited to specific embodiments to be described hereinafter but should be understood as including a variety of modifications, equivalents, and alternatives within the spirit and scope of the present disclosure. Rather, these embodiments are provided so that the description of the present disclosure will be complete and will fully convey the scope of the present disclosure to a person having ordinary skill in the art in the technical field to which the present disclosure pertains. In the following description of the present disclosure, a detailed description of related known technology will be omitted when the description may render the subject matter of the present disclosure unclear.

The terminology used in this application is used to describe specific embodiments only and is not intended to limit the invention. Expressions in the singular include the plural unless the context clearly indicates otherwise.

In the present application, the terms "includes" or "has" and the like are intended to designate the presence of the features, numbers, steps, actions, components, parts, or combinations thereof described in the specification, and are not intended to preclude the possibility of the presence or addition of one or more other features, numbers, steps, actions, components, parts, or combinations thereof. Terms such as first, second, and the like may be used to describe various components, but the components are not to be limited by such terms. Such terms are used only to distinguish one component from others

Hereinafter, embodiments according to the present disclosure will be described in detail with reference to the accompanying drawings, wherein identical or corresponding components are given the same reference numerals and duplicate descriptions thereof are omitted.

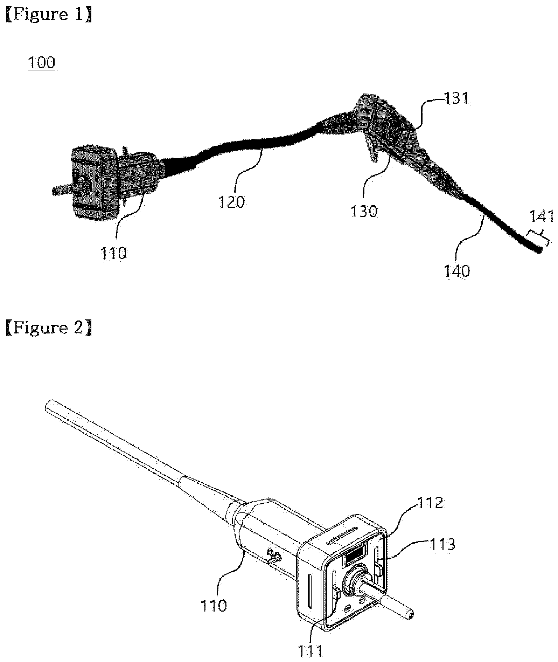

FIG. 1 illustrates the shape of an endoscope according to an embodiment of the present disclosure. FIG. 2 illustrates the shape of the connector of the endoscope according to an embodiment of the present disclosure.

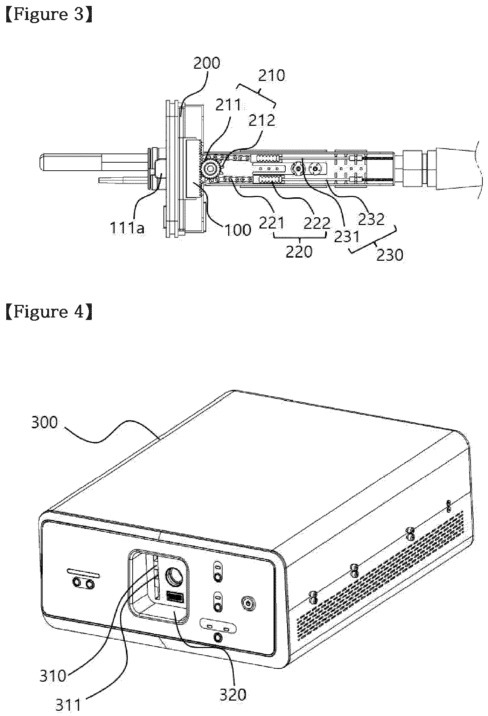

FIG. 3 illustrates the internal inside shape of the connector of FIG. 2 from which the cover is removed, i.e., the power receiving part and the power transmission part, and FIG. 4 illustrates the shape of a light source device to which the endoscope according to an embodiment of the present disclosure is coupled.

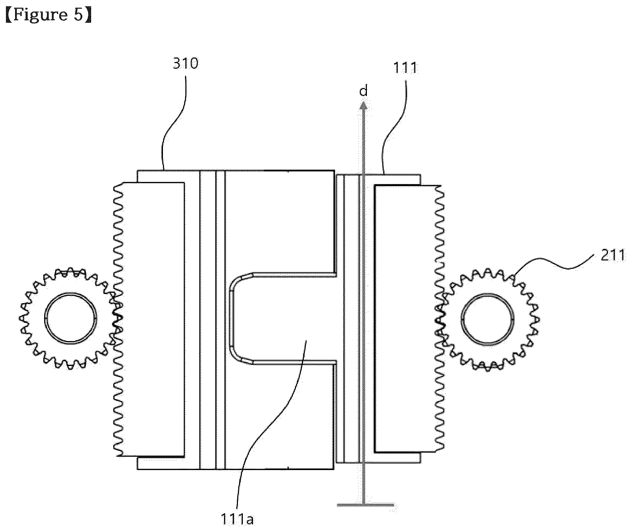

FIG. 5 illustrates the combined shape of the power providing part disposed on the light source device of FIG. 4 and the power receiving part disposed on the connector of FIG. 3.

In some embodiments, an endoscope 100 according to an embodiment of the present disclosure may include one or more among an insertion tube 140, a control body 130, and a universal cord 120, and a connector 110.

In some embodiments, the control body 130 may be disposed between one end of the insertion tube 140 and one end of the universal cord 120, and the connector 110 may be connected to the other end of the universal cord 120.

The insertion tube 140 may be a portion that is inserted into the patient's body when a user, a doctor, performs an endoscopy on the patient using the endoscope 100. The insertion tube 140 may include a tube tip, a bendable section 141, and a flexible portion.

The tube tip may be a component configured to illuminate a target area, collect image information, perform a treatment, or the like. The tube tip may be disposed on the distal end of the insertion tube, and may include an illumination means for illuminating the interior of the subject's body, an imaging means for imaging the interior of the subject's body, a biopsy channel for collecting tissue from the interior of the subject's body, an air-water channel for spraying air, water, or the like for various purposes, and the like.

The bendable section 141 performs a bending motion in response to the user's operation, and may be bent inside the body and travel along the interior of the curved tubular organ. The tube tip may be disposed on the distal end of the bendable section 141, and the distal end of the bendable section 141 may bend in the bending motion to place the tube tip in a direction desired by the user.

The flexible portion may be located between the bendable section 141 and the control body 130, and may be a portion that moves along with the bendable section 141 when the bendable section travels through the gastrointestinal tract inside the patient's body.

The control body 130 may be provided with a controller 131 to control the bending motion of the bendable section 141, and may be provided with a flow control valve or a flow control switch able to control a flow of air or water or suction. The controller 131 may include, for example, a joystick.

The insertion tube 140 may be connected to one side of the control body 130, and the universal cord 120 may be connected to the other side of the control body 130. The connector 110 may be connected to the distal end of the universal cord 120.

The connector 110 serves to connect the endoscope 100 to an external device. Here, the external device may include, for example, a light source device 300, an image processing device, and the like.

When the endoscope 100 is connected to the light source device 300 or the image processing device via the connector 110, the endoscope 100 may receive light from the light source device 300 via the connector 110 and illuminate the interior of the patient's body, and the image information regarding the interior of the patient's body collected by the endoscope 100 may be transmitted to the image processing device via the connector 110 and the light source device 300. In some embodiments, image information may be transmitted to the image processing device via the connector.

The universal cord 120 may connect the control body 130 and the connector 110, and may serve to space the connector 110 and the control body 130 apart from each other so that a user can move easily when using the endoscope 100 by holding the control body 130. In some embodiments, the universal cord 120 may be omitted and the connector 110 may be connected to the other side of the control body 130.

The endoscope 100 includes a power receiving part 111 configured to move by receiving power from a power source; and a bendable section 141, the bending motion of which is controlled by the movement of the power receiving part 111. In some embodiments, the bendable section 141 may include an imaging means for being inserted into the subject's body to collect image information therefrom and an illumination means for illuminating the interior of the subject's body.

Here, the power source may refer to a power generating device and may include, for example, a motor. In some embodiments, the power source may be disposed in the endoscope 100. In some embodiments, the power source may be disposed outside the endoscope 100. The power source disposed outside the endoscope 100 may refer to the power source being disposed on a power providing device provided separately from the endoscope 100. In some embodiments, the power providing device may include, for example, the light source device 300 or an image processing device. In this case, the endoscope 100 may be coupled to the light source device 300 or the image processing device to move by receiving power from the light source device 300 or the image processing device.

In some embodiments, when the endoscope 100 according to the present embodiment is connected to the light source device 300 having the power source therein, the endoscope 100 may illuminate the interior of the patient's body by receiving light from the light source device 300 and perform the bending motion inside the patient's body by receiving power from the light source device 300

In addition, when the endoscope 100 is connected to the image processing device including a power source therein, the endoscope 100 may transmit collected image information collected from inside the patient's body to the image processing device. The endoscope 100 may perform the bending motion inside the patient's body by receiving power from the image processing device.

The power receiving part 111 according to the present embodiment may refer to a power receiving component. The power receiving part 111 may include a mechanical power receiving component.

In some embodiments, the power receiving part 111 may receive power from an external device present outside the endoscope 100 as an article separate from the endoscope 100. In some embodiments, the power receiving part 111 may receive power directly from the light source device 300 including the power source therein.

The power receiving part 111 according to the invention is disposed on the connector 110 of the endoscope 100. When the connector 110 of the endoscope 100 is connected to the light source device 300 including the power source therein, the connector 110 may receive power from the power source of the light source device 300.

In some embodiments, a power providing part 310 supplying power to the light source device 300 may be disposed. The power providing part 310 disposed on the light source device 300 may be formed to correspond to the shape of the power receiving part 111 disposed on the connector 110. In some embodiments, the power providing part 310 may be provided with recesses 311. The power receiving part 111 is provided with protrusions 111a. The protrusions 111a of the power receiving part 111 have a structure protruding outward through slots 113 formed in a front cover 112 of the connector 110.

When the connector 110 is coupled to a connector receptacle 320 of the light source device 300, the power providing part 310 and the power receiving part 111 may be coupled to each other and be ready for transmitting power from the light source device 300 to the connector 110.

There may be a variety of methods of transmitting power from the light source device 300 to the endoscope 100. In some embodiments, the power providing part 310 may have a configuration like a slider coupled to a rail structure provided on the light source device 300 to move on the rail structure. The power receiving part 111 also has a configuration like a slider coupled to a rail structure 200 provided on the connector 110 to move on the connector 110, in the same manner as the power providing part 310.

In this structure, when the power providing part 310 moves by receiving power from the power source, the power receiving part 111 engaged with the power providing part 310 may move along with the power providing part 310, so that power may be provided by the light source device 300 to the connector 110.

The endoscope 100 according to the present embodiment may sequentially include the connector 110, the universal cord 120, the control body 130, and the insertion tube 140. Here, mechanical cords 230 may be disposed to sequentially extend through the connector 110, the universal cord 120, the control body 130, and the insertion tube 140. One side of each of the mechanical cords 230 may be connected to the power receiving part 111, and the other side of each of the mechanical cord 230 may be connected to the bendable section 141 forming one end of the insertion tube 140.

The power receiving part 111 may be connected to the mechanical cords 230 through a power transmission part. The power transmission part may include a pinion-sprocket assembly 210 and a chain-slider assembly 220.

In some embodiments, the pinion-sprocket assembly 210 may have a structure in which a pinion gear 211 and a sprocket 212 are integrally provided on a single rotating structure such that when the pinion gear 211 rotates, the sprocket 212 also rotates together. The chain-slider assembly 220 may have a structure in which a pair of sliders 222 are coupled to both ends of a chain 221, respectively, such that when the chain 221 moves, the sliders 222 connected to both ends of the chain 221, respectively, also move.

A rack gear may be provided on the rear surface of the power receiving part 111. The pinion gear 211 of the pinion-sprocket assembly 210 may be meshed with the rack gear such that power may be transmitted. In addition, in the pinion-sprocket assembly 210, the sprocket 212 may be meshed with the chain 221 of the chain-slider assembly 220 such that power may be transmitted.

The mechanical cords 230 may be connected to the paired sliders 222 of the chain-slider assembly 220, respectively. In this case, a first cord 231 and a second cord 232 described below may be connected to the paired sliders 222, respectively. Due to this structure, the first cord 231 and the second cord 232 may be paired.

According to the above-described configuration of the power transmission part, when the power receiving part 111 slides, the mechanical cords 230 are pulled or pushed to transmit power to the bendable section 141, and the bendable section 141 performs the bending motion by receiving power through the mechanical cords 230. Consequently, the bending motion of the bendable section 141 may be controlled by the movement of the power receiving part 111.

In some embodiments, the control body 130 may generate a control signal. The control signal generated by the control body 130 may control the rotational force of the power source. In some embodiments, the control signal generated by the control body 130 may control the travel distance d of the power receiving part 111.

The control body 130 according to the present embodiment may be a component having dimensions and a shape by which the user may hold the control body 130 to insert the insertion tube 140 including the bendable section 141 into the interior of the subject's body or rotate the same, and may serve as a type of handle. The user may collect image information or perform a treatment inside the subject's body by adjusting the position of the bendable section 141 inside the subject's body while holding the control body 130.

In some embodiments, the control body 130 may be provided with a controller 131, for example, a control stick, generating a control signal. The control signal generated by the controller 131 may include a control command for controlling the rotational force of the power source.

When the power source is disposed on an external device, for example, the light source device 300, the control signal may be transmitted from the endoscope 100 to an external device via a wired or wireless medium.

In some embodiments, when the control signal is transmitted via a wireless medium, in an embodiment for realizing wireless transmission, a communication module may be mounted on the control body 130, and the rotational force of the power source may be controlled by transmitting the control signal to a power source controller by the communication module.

In some embodiments, when the control signal is transmitted via a wired medium, in an embodiment for realizing wired transmission, an electrical cable configuration may be connected to the control body 130, the universal cord 120, and the connector 110 in a control stick, and a control signal terminal may be disposed on one end of the connector 110.

In addition, in this case, the connector receptacle 320 of the light source device 300 may also be provided with a control signal receiving terminal, and the control signal receiving terminal may have a structure electrically connected to the power source controller.

When the connector 110 is connected to the light source device 300, the control signal terminal may be in contact with the control signal receiving terminal, and a control signal generated by the control stick may be transmitted to the power source controller through an electrical cable, the control signal terminal, and the control signal receiving terminal.

In some embodiments, a control signal generated by the controller 131 disposed on the control body 130 may control the travel distance d of the power providing part 310 of the light source device 300 or the power receiving part 111 of the connector 110.

That is, the control signal generated by the control body 130 may control the travel distance d of the power receiving part 111 by a structure in which the control signal controls the rotational force of the power source of the light source device 300, the power source generates power, and the generated power is sequentially transmitted through the power providing part 310 and the power receiving part 111.

In some embodiments, the direction of the rotation of the power source may determine the direction of the movement of the power providing part 310 or the power receiving part 111, and the speed of the rotation of the power source may determine the speed of the movement of the power providing part 310 or the power receiving part 111. In addition, the direction of the movement of the power receiving part 111 may determine the direction of the bending of the bendable section 141, and the speed of the movement of the power receiving part 111 may determine the speed of the bending of the bendable section 141.

The user, i.e., a doctor, may generate the control signal by operating the control stick and transmit the generated control signal to the power source disposed on the light source device 300. The power source that has received the control signal may generate rotational force in response to the control signal. The power providing part 310 and the power receiving part 111 may move in response to the generated rotational force. Consequently, the bending motion of the bendable section 141 may be controlled by the movement of the power receiving part 111.

According to the invention, the power receiving part 111 includes a type of slider. The power receiving part 111 is coupled to the rail structure 200 provided on the connector 110 to move in a specific direction on the rail structure 200. The power receiving part 111 reciprocally slides between a first end and a second end formed by the rail structure 200 provided on the connector 110.

For example, the power receiving part 111 may move within a range of bending angles (i.e., a bending angle range) of the bendable section 141 while reciprocating between the first end and the second end. Here, the first end of the power receiving part 111 may correspond to a first angle of the bending angle range, and the second end of the power receiving part 111 may correspond to a second angle of the bending angle range. For example, when the power receiving part 111 moves from the first end to the second end, the bendable section 141 may be bent from the first angle to the second angle.

The bending angle range of the bendable section 141 may be previously determined to a set value. The bending angle range of the bendable section 141 may be set such that the bending is limited, for example, between the first angle and the second angle. Accordingly, the position of the bendable section 141 may be accurately controlled by a computer through electrification of the endoscope 100, and the user, i.e., a doctor, may estimate the range of the bending of the bendable section 141.

The control signal may allow the bending motion of the bendable section 141 to precisely conform to the predetermined bending angle range by controlling the travel distance d of the power providing part 310 disposed on the light source device 300 or the power receiving part 111 disposed on the connector 110.

In some embodiments, the power receiving part 111 may include a first slider and a second slider, and the control signal may include a first control signal for controlling the travel distance d of the first slider and a second control signal for controlling the travel distance d of the second slider.

The power receiving part 111 may include a pair of components. That is, the power receiving part 111 may include a first slider and a second slider. The power providing part 310 may include a pair of components like the power receiving part 111. In the control signal, he first control signal may control the travel distance d of the first slider, and the second control signal may control the travel distance d of the second slider.

In some embodiments, the mechanical cords 230 transmitting power to the bendable section 141 may include the first cord 231, the second cord 232, a third cord, and a fourth cord.

In some embodiments, the first cord 231, the second cord 232, the third cord, and the fourth cord may be controlled by four sliders that move independently of each other.

In addition, in other embodiments, the first cord 231 and the second cord 232 may form a first set of cords, and the third cord and the fourth cord may form a second set of cords.

In the first set of cords, the first cord 231 and the second cord 232 have a paired structure in which when the first cord 231 is pulled, the second cord 232 is pushed and when the second cord 232 is pulled, the first cord 231 is pushed. In the second set of cords, the first cord 231 and the second cord 232 have a paired structure in which when first cord 231 is pulled, the second cord 232 is pushed and when the second cord 232 is pulled, the first cord 231 is pushed.

Here, the first set of cords may be connected to the first slider to transmit power in response to the linear movement of the first slider, and the second set of cords may be connected to the second slider to transmit power in response to the linear movement of the second slider. According to the configuration in which the power receiving part 111 according to the present embodiment includes the first slider and the second slider, two sets of cords in which every two mechanical cords 230 are paired may be respectively controlled in an effective manner.

In some embodiments, the power receiving part 111 may be a pair of power receiving parts. One of the pair of power receiving parts may control the upward and downward bending of the bendable section 141, and the other of the pair of power receiving parts may control the left and right bending of the bendable section 141.

In the pair of power receiving parts 111, one power receiving part 111 may control the upward and downward bending motion of the bendable section 141, while the other power receiving part 111 may control the left and right bending motion of the bendable section 141.

According to this structure, one and the other of the pair of power receiving parts 111 may move linearly and independently of each other. Due to the combination of the linear movements of the pair of power receiving parts 111, the upward and downward bending and the left and right bending of the bendable section 141 may be combined, thereby realizing the bending motion in upward-downward and left-right directions.

In some embodiments, a first range of bending angles between the minimum angle and the maximum angle in which the upward and downward bending motion is performed may differ from a second range of bending angles between the minimum angle and the maximum angle in which the left and right bending motion is performed. The difference between the first range of bending angles and the second range of bending angles is intended to represent a reference point to a user, i.e., a member of medical staff, when the user is controlling the bending motion by combining the upward and downward bending motion and the left and right bending motion, so that the user may easily estimate the position of the tube tip located inside the patient's body during an endoscopy. That is, the range of bending angles in which the bendable section is bent in the upward and downward directions by one of the pair of power receiving parts 111 and the range of bending angles in which the bendable section is bent in the left and right directions by the other of the pair of power receiving parts 111 may differ from each other, so that the user, i.e., a member of medical staff, may easily estimate the position of the tube tip of the distal end of the bendable section.

In some embodiments, the pair of power receiving parts 111 may include a first slider and a second slider. The first slider may control the upward and downward bending motion of the bendable section 141, and the second slider may control the left and right bending motion of the bendable section 141.

According to this structure, the first slider and the second slider may move linearly and independently of each other. Due to the combination of the linear movements of the first slider and the second slider, the upward and downward bending and the left and right bending of the bendable section 141 may be combined, thereby realizing the bending motion in upward-downward and left-right directions. The first slider and the first slider may be components corresponding to one and the other of the pair of power receiving parts 111, respectively.

According to another embodiment of the present disclosure, the endoscope 100 may include one or more among: an insertion tube 140 including a bendable section 141 configured to be inserted into a subject's body in order to collect image information;
a control body 130 configured to generate a control signal to control a bending angle of the bendable section 141; and
a connector 110 including an output part configured to output the control signal and a receiving part configured to receive external power corresponding to the control signal, wherein the control signal controls the travel distance of the receiving part. Since the insertion tube 140, the control body 130, and the connector 110 have already been described in the foregoing embodiment, a detailed description thereof will be omitted.

In the specification of the present disclosure, the use of the term "the or said" and similar denoting terms may correspond to both singular and plural forms. Furthermore, recitation of ranges of values herein are merely intended to refer to respective separate values falling within the respective ranges and, unless otherwise indicated herein, the respective separate values are incorporated herein as if individually recited herein.

The operations of any method described herein may be performed in any suitable order unless otherwise indicated herein or otherwise clearly contradicted by the context. However, the operations shall not be limited to the described sequence. The use of any examples or exemplary languages (e.g., "such as") provided herein, is intended merely to better illustrate the present disclosure and does not pose a limitation on the scope of the present disclosure unless otherwise defined by the Claims.

### <Description of Reference Numerals of Drawings>

100: endoscope
110: connector
111: power receiving part
111a: protrusion
112: front cover
113: slot
120: universal cord
130: control body
131: controller
140: insertion tube
141: bendable section
200: rail structure
210: pinion-sprocket assembly
211: pinion gear
212: sprocket
220: chain-slider assembly
221: chain
222: slider
230: mechanical cord
231: first cord
232: second cord
300: light source device
310: power providing part
311: recess
320: connector receptacle
d: travel distance

## Claims

1. An endoscope (100) comprising:
a connector (110) connected to an external device having a power source and the connector configured to receive power;
a power receiving part (111) disposed on the connector and configured to move by receiving power from the power source; and
a bendable section (141), a bending motion of which is controlled by a movement of the power receiving part for being bent inside the body and traveling along the interior of a curved tubular organ,
wherein the power receiving part has protrusions (111a) protruding outward through slots (113) formed in a front cover (112) of the connector and wherein the protrusions connected to the external device are configured to receive power,
**characterized in that**
the connector further comprises a rail structure (200) and **in that** the power receiving part further comprises a slider coupled to the rail structure provided on the connector and
wherein the power receiving part is configured tc reciprocally slide between a first end and a second end on the rail structure such that when the power receiving part moves from the first end to the second end, the bendable section is bent from a first angle to a second angle.

2. The endoscope of claim 1, wherein the bendable section comprises an imaging means configured to be inserted into a subject's body to collect image information and an illumination means configured to illuminate an interior of the subject's body.

3. The endoscope of claim 1, further comprising a control body configured to generate a control signal, wherein the control signal generated by the control body controls rotational force of the power source.

4. The endoscope of claim 1, further comprising a control body configured to generate a control signal, wherein the control signal generated by the control body controls a travel distance of the power receiving part.

5. The endoscope of claim 4, wherein the power receiving part comprises a first slider and a second slider, and the control signal comprises a first control signal to control a travel distance of the first slider and a second control signal to control a travel distance of the second slider.

6. The endoscope of claim 4, wherein the power receiving part comprises a pair of power receiving parts, wherein one of the pair of power receiving parts controls upward and downward bending of the bendable section and the other of the pair of power receiving parts controls left and right bending of the bendable section.

7. The endoscope of claim 4, wherein power generated due to the movement of the power receiving part is transmitted to the bendable section through the control body.

8. The endoscope of claim 1, wherein the power receiving part receives power directly from an external device comprising the power source therein.

## Patentansprüche

1. Endoskop (100), umfassend:
einen Verbinder (110), der mit einer externen Vorrichtung verbunden ist, die eine Stromquelle aufweist, und der Verbinder so konfiguriert ist, dass er Strom aufnimmt;
ein Stromaufnahmeteil (111), das sich an dem Verbinder befindet und so konfiguriert ist, dass es sich durch das Aufnehmen von Strom von der Stromquelle bewegt; und
einen biegbaren Abschnitt (141),
dessen Biegebewegung durch eine Bewegung des Stromaufnahmeteils gesteuert wird, um innerhalb des Körpers gebogen zu werden und sich entlang des Inneren eines gekrümmten röhrenförmigen Organs zu bewegen,
wobei das Stromaufnahmeteil Vorsprünge (111a) aufweist, die durch Schlitze (113), die in einer vorderen Abdeckung (112) des Verbinders ausgebildet sind, nach außen ragen, und wobei die mit der externen Vorrichtung verbundenen Vorsprünge so konfiguriert sind, dass sie Strom aufnehmen,
**dadurch gekennzeichnet, dass**
der Verbinder ferner eine Schienenstruktur (200) umfasst und dass das Stromaufnahmeteil ferner einen Schieber umfasst, der mit der an dem Verbinder vorgesehenen Schienenstruktur gekoppelt ist, und
wobei das Stromaufnahmeteil so konfiguriert ist, dass es zwischen einem ersten Ende und einem zweiten Ende auf der Schienenstruktur hin und her gleitet, so dass, wenn sich das Stromaufnahmeteil von dem ersten Ende zu dem zweiten Ende bewegt, der biegbare Abschnitt von einem ersten Winkel zu einem zweiten Winkel gebogen wird.

2. Endoskop gemäß Anspruch 1, wobei der biegbare Abschnitt ein Abbildungsmittel umfasst, das so konfiguriert ist, dass es in den Körper eines Subjekts eingeführt wird, um Bildinformationen zu sammeln, und ein Beleuchtungsmittel, das so konfiguriert ist, dass es ein Inneres des Körpers des Subjekts beleuchtet.

3. Endoskop gemäß Anspruch 1, ferner umfassend einen Steuerkörper, der so konfiguriert ist, dass er ein Steuersignal erzeugt, wobei das von dem Steuerkörper erzeugte Steuersignal die Rotationskraft der Stromquelle steuert.

4. Endoskop gemäß Anspruch 1, ferner umfassend einen Steuerkörper, der so konfiguriert ist, dass er ein Steuersignal erzeugt, wobei das von dem Steuerkörper erzeugte Steuersignal einen Verfahrweg des Stromaufnahmeteils steuert.

5. Endoskop gemäß Anspruch 4, wobei das Stromaufnahmeteil einen ersten Schieber und einen zweiten Schieber umfasst, und das Steuersignal ein erstes Steuersignal zum Steuern eines Verfahrwegs des ersten Schiebers und ein zweites Steuersignal zum Steuern eines Verfahrwegs des zweiten Schiebers umfasst.

6. Endoskop gemäß Anspruch 4, wobei das Stromaufnahmeteil ein Paar von Stromaufnahmeteilen umfasst, wobei eines des Paars von Stromaufnahmeteilen das Aufwärts- und Abwärtsbiegen des biegbaren Abschnitts steuert und das andere des Paars von Stromaufnahmeteilen das Links- und Rechtsbiegen des biegbaren Abschnitts steuert.

7. Endoskop gemäß Anspruch 4, wobei der aufgrund der Bewegung des Stromaufnahmeteils erzeugte Strom durch den Steuerkörper auf den biegbaren Abschnitt übertragen wird.

8. Endoskop gemäß Anspruch 1, wobei das Stromaufnahmeteil Strom direkt von einer externen Vorrichtung aufnimmt, die die Stromquelle darin umfasst.

## Revendications

1. Un endoscope (100) comprenant :
un connecteur (110) connecté à un dispositif externe doté d'une source d'énergie et le connecteur configuré pour recevoir de l'énergie ;
une partie de réception d'énergie (111) disposée sur le connecteur et configurée pour se déplacer en recevant de l'énergie de la source d'énergie ; et
une section pliable (141),
dont un mouvement de flexion est contrôlé par un mouvement de la partie réceptrice d'énergie pour être pliée à l'intérieur du corps et se déplacer le long de l'intérieur d'un organe tubulaire incurvé,
dans lequel la partie réceptrice d'énergie a des protubérances (111a) faisant saillie vers l'extérieur à travers des fentes (113) formées dans un couvercle avant (112) du connecteur et dans lequel les protubérances connectées au dispositif externe sont configurées pour recevoir de l'énergie,
**caractérisé par le fait que**
le connecteur comprend en outre une structure de rail (200) et la partie réceptrice d'énergie comprend en outre un curseur couplé à la structure de rail prévue sur le connecteur et
dans lequel la partie réceptrice d'énergie est configurée pour glisser réciproquement entre une première extrémité et une deuxième extrémité sur la structure de rail de sorte que lorsque la partie réceptrice d'énergie se déplace de la première extrémité à la deuxième extrémité, la section pliable est pliée d'un premier angle à un deuxième angle.

2. L'endoscope de la revendication 1, dans lequel la section pliable comprend un moyen d'imagerie configuré pour être inséré dans un corps d'un sujet afin de recueillir des informations d'image et un moyen d'éclairage configuré pour éclairer un intérieur du corps du sujet.

3. L'endoscope de la revendication 1, comprenant en outre un organe de commande configuré pour générer un signal de commande, dans lequel le signal de commande généré par l'organe de commande contrôle la force de rotation de la source d'énergie.

4. L'endoscope de la revendication 1, comprenant en outre un organe de commande configuré pour générer un signal de commande, dans lequel le signal de commande généré par l'organe de commande contrôle une distance de déplacement de la partie réceptrice d'énergie.

5. L'endoscope de la revendication 4, dans lequel la partie réceptrice d'énergie comprend un premier curseur et un second curseur, et le signal de commande comprend un premier signal de commande pour contrôler une distance de déplacement du premier curseur et un second signal de commande pour contrôler une distance de déplacement du second curseur.

6. L'endoscope de la revendication 4, dans lequel la partie réceptrice d'énergie comprend une paire de parties réceptrices d'énergie, dans laquelle l'une de la paire de parties réceptrices d'énergie contrôle la flexion vers le haut et vers le bas de la section pliable et l'autre de la paire de parties réceptrices d'énergie contrôle la flexion vers la gauche et vers la droite de la section pliable.

7. L'endoscope de la revendication 4, dans lequel de l'énergie générée par le mouvement de la partie réceptrice d'énergie est transmise à la section pliable par l'intermédiaire du corps de commande.

8. L'endoscope de la revendication 1, dans lequel la partie réceptrice d'énergie reçoit de l'énergie directement d'un dispositif externe comprenant la source d'énergie dans celui-ci.
